# EUROPÄISCHE PATENTSCHRIFT

(11) **EP 1 967 227 B1**
(45) Veröffentlichungstag und Bekanntmachung des Hinweises auf die Patenterteilung: **19.12.2012**
(21) Anmeldenummer: 07103693.3
(22) Anmeldetag: 07.03.2007
(51) Int. Cl.: A61N 5/06

(54) **Bestrahlungsgerät zur Bestrahlung eines menschlichen Körpers**
Radiation device for irradiating a human body
Dispositif d'irradiation destiné à l'irradiation d'un corps humain

(43) Veröffentlichungstag der Anmeldung: 10.09.2008
(73) Patentinhaber: Flowil International Lighting (HOLDING) B.V., 1097 JB Amsterdam (NL)
(72) Erfinder: Smolka, Ernst, 63773 Goldbach (DE)
(74) Vertreter: Murnane, Graham John

(56) Entgegenhaltungen:
- EP-A- 0 214 353
- DE-A1- 3 413 662
- DE-A1- 4 312 547
- DE-U1- 9 313 669
- DE-U1-202005 002 918
- US-A- 4 469 951

## Beschreibung

Die Erfindung betrifft ein Bestrahlungsgerät zur Bestrahlung eines menschlichen Körpers, speziell zur Ganzkörperbestrahlung, mit mindestens zwei Typen röhrenförmiger UVA und/oder UVB-Strahlung abgebender Niederdruckstrahler sowie ein Verfahren zum Betreiben eines Bestrahlungsgerätes zur Ganzkörperbestrahlung, speziell eines menschlichen Körpers, mit mindestens zwei Typen röhrenförmiger, UVA und/oder UVB-Strahlung abgebender Niederdruckstrahler.

Herkömmliche Bestrahlungsgeräte, insbesondere Solarien werden in verschiedene Bestrahlungsklassen eingeteilt, wobei eine Haupteinteilung dadurch bedingt ist, dass bei einer ersten Gruppe von Bestrahlungsgeräten die UVA-Abstrahlung bei einer zweiten Gruppe von Geräten die UVB-Abstrahlung im Vordergrund steht.

Der UVA-Bereich ultravioletter Strahlung ist durch eine Wellenlänge von 315 bis 400 nm definiert. Der kurzwelligere Bereich der ultravioletten UVB-Strahlung liegt im Bereich von 280 bis 315 nm.

Bei der herkömmlich gegebenen Aufteilung von Bestrahlungsgeräten in unterschiedliche Klassen müssen die Hersteller von Bestrahlungsgeräten eine Vielzahl unterschiedlicher Geräte produzieren, was zu aufwändigen Produktionsabläufen, aufwändiger Lagerhaltung, etc. führt. Die Betreiber von Sonnenstudios müssen ebenfalls eine Mehrzahl unterschiedlicher Typen von Bestrahlungsgeräten vorhalten, um je nach Anwendungsfall den unterschiedlichen Vorgaben gerecht zu werden.

Ein Strahlenspektrum eines bestimmten Niederdruckstrahlers wird nämlich dadurch erreicht, dass mehrere Leuchtstoffe vermischt werden. Diese emittieren bei Anregung, die in bekannter Weise in der Regel durch Hg-Strahlung vorgenommen wird, in unterschiedlichen Wellenlängen. Der Bedarf an unterschiedlichen Spektren mit einem jeweils festgelegten Verhältnis zwischen UVA-Strahlung und UVB-Strahlung ist groß. Daraus sind viele Lampentypen entstanden, was hohe Kosten im Herstellungsprozess, bei der Lagerung, sowie in der Logistik, wie bereits angesprochen, erzeugt.

Die DE 93 13 669 U1 offenbart eine Vorrichtung zum Bräunen des menschlichen Körpers, die zum Verständnis der vorliegenden den Erfindung nützlich sein kann.

Auch wurden im Stand der Technik bereits Bestrahlungsgeräte vorgeschlagen, bei denen die Bestrahlungsleistung in ihren UVA/UVB-Anteil einstellbar ist. Ein Beispiel für ein solches Bestrahlungsgerät geht aus der DE 78 26 093 U1 hervor.

Dort sind in einem Strahlungsfeld eine Mehrzahl von Niederdruckstrahlern eines ersten Typs A sowie Niederdruckstrahlern eines zweiten Typs B angeordnet sind, wobei die Niederdruckstrahler des zweiten Typs B gegenüber den Niederdruckstrahlern des ersten Typs A eine höhere UVB-Abstrahlung aufweisen. Die dort konkret vorgeschlagene Lösung sieht vor, zwischen Gruppen von zwei oder drei Niederdruckstrahlern des ersten Typs A jeweils einen Niederdruckstrahler des zweiten Typs B anzuordnen. Dabei sind die Abstände der Niederdruckstrahler untereinander vergleichsweise groß. Weiter sind die Durchmesser der Niederdruckstrahler des ersten Typs A zu den Durchmessern der Niederdruckstrahler des zweiten Typs B identisch.

Die Aufgabe der vorliegenden Erfindung besteht demgegenüber darin, ein Bestrahlungsgerät sowie ein entsprechendes Verfahren vorzuschlagen, bei dem sowohl UVA- als auch UVB-Strahlung abgegeben werden kann und das Verhältnis von UVA- und UVB-Strahlung bei möglichst gleichmäßiger flächenmäßiger Verteilung der Strahlungsleistung eingestellt werden kann.

Diese Aufgabe wird gelöst mit einem Bestrahlungsgerät nach den Merkmalen des Anspruches 1 bzw. mit einem Verfahren nach den Merkmalen des Anspruches 13.

Die erfindungsgemäße alternierende Anordnung von Niederdruckstrahlern des ersten Typs A sowie von Niederdruckstrahlern des zweiten Typs B kann entweder über das komplette Bestrahlungsfeld eines Bestrahlungsgerätes oder über Teilbereiche des Bestrahlungsgerätes, z. B. nur in der Mitte bzw. unter Aussparung eines Gesichtsbräuners vorgesehen werden.

Vorteilhafte Weiterbildungen sind in den Unteransprüchen angegeben.

Die Erfindung schlägt somit ein Bestrahlungsgerät vor, das sich in mehreren Aspekten deutlich vom eingangs beschriebenen Stand der Technik unterscheidet. Zum Einen werden die Niederdruckstrahler des ersten Typs A und die Niederdruckstrahler des zweiten Typs B alternierend angeordnet und vor allem die Niederdruckstrahler des zweiten Typs B mit geringerem Durchmesser ausgestaltet, so dass die von den Niederdruckstrahlern des ersten Typs A erzeugte Abstrahlung möglichst wenig in ihrer Homogenität und UV-Leistung gestört wird bzw. die Niederdruckstrahler des ersten Typs A möglichst wenig voneinander beabstandet sind.

Mit einem Bestrahlungsgerät nach dem eingangs diskutierten Stand der Technik war mit dem dort vorgeschlagenen Aufbau eine gleichmäßige Bestrahlung sowohl im UVA- als auch im UVB-Bereich nicht zu erzielen. Demgegenüber hat die Anmelderin erkannt, dass beim Bestrahlungsfeld die Niederdruckstrahler des ersten Typs A möglichst gleichmäßig angeordnet werden sollen, wobei bereits die Einfügung eines Niederdruckstrahlers mit anderem Abstrahlspektrum gleichen Durchmessers diese Gleichmäßigkeit empfindlich stört, so dass Niederdruckstrahler mit geringerem Durchmesser vorgeschlagen werden.

Solarien werden derzeit oftmals mit T12-Niederdruckstrahlern bestückt, die einen Durchmesser von 12/8 Inch = 38,1 mm aufweisen. Würde man einzelne T12-Niederdruckstrahler des ersten Typs A durch Niederdruckstrahler des zweiten Typs B ersetzen, würden sich zu große Abstände zwischen den Niederdruckstrahlern des ersten Typs A ergeben. Nach einer Überlegung der vorliegenden Erfindung sind daher die für die UVB-Abstrahlung vorwiegend verantwortlichen Niederdruckstrahler mit geringerem Durchmesser ausgebildet. Bei der Verwendung von T12-Niederdruckstrahlern für den Typ A kommen erfindungsgemäß T5-Niederdruckstrahler zum Einsatz. Die Niederdruckstrahler des zweiten Typs B, insbesondere die T5-Niederdruckstrahler, weisen eine Länge ≥ 120 cm, vorzugsweise ≥ 180 cm auf, so dass sich die T5-Niederdruckstrahler jeweils über die gesamte Länge des Bestrahlungsfeldes erstrecken und die an sich benötigte Gesamtlänge von 180 cm nicht durch Aneinandersetzen von zwei kürzer bemessenen Niederdruckstrahlern erzielt werden muss, was einen höheren Montageaufwand bedeutet, da entsprechend mehr Lampen vorgehalten werden müssen, die Installation sowie die Ansteuerung aufwendiger sind.

Um die gleichmäßige Abstrahlung noch zu verbessern, sollten die Niederdruckstrahler des ersten Typs A und die Niederdruckstrahler des zweiten Typs B im Bestrahlungsfeld dicht aneinander liegen, insbesondere nicht mehr als ein viertel Lampendurchmesser der Niederdruckstrahler des ersten Typs A, weiter bevorzugt nicht mehr als ein viertel des Lampendurchmessers der Niederdruckstrahler des zweiten Typs B beabstandet sein.

Nach einem Aspekt der vorliegenden Erfindung sind die Niederdruckstrahler des zweiten Typs B, insbesondere die T5-Niederdruckstrahler als reine UVB-Lampen ausgebildet.

Unter "reinen" UVB-Lampen werden im Sinne der vorliegenden Anmeldung solche Lampen verstanden, bei denen die erythemwirksame Leistung im UVB-Bereich (280 bis 315 nm) bei nahezu 100%, insbesondere bei über 98,5%, bei einer ersten konkreten Leuchtstoffbeschichtung bei 99,6% sowie bei einer alternativen Leuchtstoffbeschichtung bei 99,8% liegt.

Vorzugsweise sind die Niederdruckstrahler des zweiten Typs B als reine UVB-Lampen ausgebildet.

Nach einer bevorzugten Ausgestaltung sind die Niederdruckstrahler des ersten Typs als reine UVA-Lampen ausgebildet. Unter "reinen" UVA-Lampen im Sinne der vorliegenden Erfindung werden hier Lampen mit einem UVB/UVA-Verhältnis von < 0,25% verstanden (UVA: 315 bis 400 nm).

Bei einer konkreten Ausführungsform der Niederdruckstrahler des ersten Typs A, die konkret als T12-Lampe ausgebildet waren, ergab sich für den Bereich 250 bis 320 nm eine erythemwirksame Leistung von 0,273 µW/cm² und für den Bereich 321 bis 400 nm eine erythemwirksame Leistung in Höhe von 0,532 µW/cm².

Bei einem nach einer konkreten Ausgestaltung vorliegenden Niederdruckstrahler des Typs B, der konkret als T5-Lampe ausgebildet war, ergab sich im Bereich von 250 bis 320 nm eine erythemwirksame Leistung von 18,26 µW/cm² und im Bereich von 321 bis 400 nm eine erythemwirksame Leistung in Höhe von 0,078 µW/cm².

Sämtliche Bestimmungen der erythemwirksamen Leistung wurden gemäß Cie. Puppl. 17.4 vorgenommen.

Nach einem weiter bevorzugten Aspekt der vorliegenden Erfindung ist mit der Anordnung der Niederdruckstrahler des ersten Typs A sowie der Niederdruckstrahler des zweiten Typs B sichergestellt, dass das Bestrahlungsfeld in Projektion auf die Haupt-Abstrahlrichtung im wesentlichen vollständig durch UV-Lichtquellen in Gestalt von Niederdruckstrahlern überdeckt ist. Durch diese Maßnahme der im wesentlichen vollständigen Überdeckung zusammen mit dem Grundgedanken der vorliegenden Erfindung der alternierenden Anordnung die Niederdruckstrahler des Typs A sowie des Typs B bei reduziertem Durchmesser der Niederdruckstrahler des Typs B lässt sich eine besonders gleichmäßige Strahlungsverteilung erzielen.

Nach einem bevorzugten Aspekt der vorliegenden Erfindung sind die Niederdruckstrahler des zweiten Typs B gegenüber den Niederdruckstrahlern des ersten Typs A geringfügig in Richtung der Haupt-Abstrahlrichtung versetzt, vorzugsweise nämlich so, dass sie an ihrer der Haupt-Abstrahlrichtung zugewandten Seite zumindest im wesentlichen bündig abschließen.

Mit dieser Maßnahme wird eine noch dichtere Aneinanderpositionierung der Niederdruckstrahler des ersten Typs A sowie der Niederdruckstrahler des zweiten Typs B erreicht, wobei durch den geringeren Durchmesser der Niederdruckstrahler des zweiten Typs B gleichzeitig die geringere Beabstandung der Projektion auf die Abstrahlrichtung sowie andererseits ein im wesentlichen bündiger Abschluss an der der Hauptabstrahlung zugewandten Seite erreicht wird.

Alternativ ist es aber auch möglich, die Niederdruckstrahler des zweiten Typs B gegenüber den Niederdruckstrahlern des ersten Typs A geringfügig in Richtung entgegen der Haupt-Abstrahlrichtung versetzt anzuordnen, vorzugsweise nämlich so, dass sie an ihrer der Hauptabstrahlrichtung abgewandten Seite zumindest im wesentlichen bündig zueinander abschließen. Auch hier ergeben sich die Vorteile der einerseits dichteren Aneinanderreihung und andererseits der Erzielung eines im wesentlichen bündigen Abschlusses an der der Haupt-Abstrahlrichtung abgewandten Seite.

Die Niederdruckstrahler des ersten Typs A können eine Länge von mindestens 60 cm, vorzugsweise von mindestens 150 cm, weiter vorzugsweise von mindestens 180 cm, besonders bevorzugt von mindestens 200 cm aufweisen. Gerade bei der Realisierung eines Bestrahlungsgerätes zur Ganzkörperbestrahlung können Längen von mindestens 180 cm bzw. 6 ft bzw. von mindestens 200 cm interessant sein, so dass über die gesamte Länge des Bestrahlungsfeldes durchgehende Niederdruckstrahler vorgesehen werden können.

Die Niederdruckstrahler des zweiten Typs B weisen eine Länge von mindestens 120 cm, vorzugsweise von mindestens 180 cm bzw. 6 ft, evtl. sogar von mindestens 200 cm auf. In gleicher Weise kann damit sichergestellt werden, dass die Niederdruckstrahler sich durchgehend über die gesamte Länge des Bestrahlungsfeldes (mit oder ohne integriertem Gesichtsbräuner) erstrecken können und nicht etwa zwei Niederdruckstrahler zueinander fluchtend hintereinander angeordnet werden müssen. Die Ausbildung von Niederdruckstrahlern des zweiten Typs in Gestalt von T5-Lampen mit Längen von mindestens 180 cm, insbesondere von mindestens 200 cm ist ein unabhängiger Aspekt der vorliegenden Erfindung, der auch als solches unabhängig beansprucht wird.

In einer bevorzugten Ausgestaltung werden etwa 15% bis 35%, bevorzugtermaßen etwa 30% des Bestrahlungsfeldes durch Niederdruckstrahler des zweiten Typs B überdeckt.

Nach einer ebenfalls bevorzugten Ausgestaltung werden etwa 65% bis 75%, bevorzugtermaßen etwa 70% des Bestrahlungsfeldes durch Niederdruckstrahler des ersten Typs A überdeckt.

Wie bereits erwähnt, wird auch ein Niederdruckstrahler mit einem Durchmesser von 5/8 Inch nach Art einer T5-Röhre unabhängig beansprucht, insbesondere zur Verwendung in einem wie vorstehend erläuterten Bestrahlungsgerät, der sich speziell dadurch auszeichnet, dass er ein Länge zu Durchmesser Verhältnis von 114 bis 130, vorzugsweise von etwa 125 aufweist.

Weiter kann auch im Bestrahlungsfeld noch ein Gesichtsbräuner integriert sein, der beispielsweise mehrere kurze T5-Strahler oder Hochdruck-Quarzstrahler umfasst. Der Gesichtsbräuner bzw. dessen Strahler können dann auch separat von den restlichen Niederdruckstrahlern des Bestrahlungsfeldes angesteuert und in der gewünschten Strahlenstärke eingestellt werden.

Das Bestrahlungsgerät nach der vorliegenden Erfindung kann insbesondere für die Ganzkörperbestrahlung und/oder für die kosmetische Bräunung eines menschlichen Körpers eingesetzt werden.

Je nach konkreter Verwendung des hier vorgeschlagenen Bestrahlungsgerätes können spezielle Leuchtstoffe für die Niederdruckstrahler des ersten Typs A sowie die Niederdruckstrahler des zweiten Typs B zur Anwendung gelangen. Gleiches gilt für Gläser für die Transmission bzw. eventuelle Filter. Das Bestrahlungsgerät nach der vorliegenden Erfindung sowie das vorgeschlagene Verfahren kann auch für die Lichttherapie eingesetzt werden.

Weiterhin wird auch ein Verfahren zum Betreiben eines Bestrahlungsgerätes gemäß Anspruch 13 vorgeschlagen.

Die Einstellung, insbesondere Drosselung, der Leistung der Niederdruckstrahler des ersten Typs A und/oder der Niederdruckstrahler des zweiten Typs B kann über den Niederdruckstrahlern jeweils zugeordnete Vorschaltgeräte vorgenommen werden.

Nach einem weiteren bevorzugten Aspekt der vorliegenden Erfindung wird die Einstellung der UVA- Leistung der Niederdruckstrahler des ersten Typs A und/oder UVB-Leistung der Niederdruckstrahler des zweiten Typs B in Abhängigkeit des Hauttyps einer zu bestrahlenden Person vorgenommen.

Die Ermittlung des Hauttyps einer zu bestrahlenden Person kann auch durch spezielle Messgeräte vorgenommen werden. Anhand der Ergebnisse eines solchen Messverfahrens kann das Bestrahlungsgerät dann eingestellt bzw. das Verfahren zum Betreiben des Bestrahlungsgerätes konkret festgelegt werden.

Nach einem weiteren bevorzugten Aspekt der vorliegenden Erfindung wird die Ermittlung des Hauttyps einer zu bestrahlenden Person automatisch ermittelt und besonders bevorzugt auch automatisch an das Bestrahlungsgerät weitergegeben bzw. automatisch für die Auswahl der Parameter des erfindungsgemäßen Verfahrens genutzt. Insbesondere kann ein Messgerät zur Ermittlung des Hauttyps einer zu bestrahlenden Person auch im Bestrahlungsgerät integriert sein.

Mit den beiden letztgenannten Überlegungen lässt sich das hier vorgeschlagene Bestrahlungsgerät besonders effizient einsetzen, da die Bestrahlung nun angepasst auf die jeweils speziellen Erfordernisse mit einem einzigen Bestrahlungsgerät durchgeführt werden kann.

Mit dem vorgeschlagenen Bestrahlungsgerät bzw. dem vorgeschlagenen Verfahren kann das erwünschte Bestrahlungsspektrum in UVA und UVB individuell eingestellt werden von nur UVA, über jedes Mischverhältnis von A und B bis zu nur UVB. Darüber hinaus kann die Bestrahlungsstärke von A und B durch die Lampenleistung eingestellt werden.

Wie bereits eingangs geschildert, müssen bei der herkömmlich gegebenen Aufteilung von Bestrahlungsgeräten unterschiedlicher Klassen die Hersteller eine Vielzahl unterschiedlicher Geräte produzieren, was zu aufwendigen Produktionsabläufen, aufwendiger Lagerhaltung etc. führt. Die Betreiber von Sonnenstudios müssen ebenfalls eine Mehrzahl unterschiedlicher Typen von Bestrahlungsgeräten vorhalten, um je nach Anwendungsfall den unterschiedlichen Vorgaben gerecht zu werden. Dies lässt sich mit dem Bestrahlungsgerät nach der Erfindung bzw. dem erfindungsgemäßen Verfahren mit einfacher Software durch lediglich ein einziges Gerät realisieren.

Die Erfindung wird nachstehend auch hinsichtlich weiterer Merkmale und Vorteile anhand der Beschreibung von Ausführungsbeispielen und unter Bezugnahme auf die beiliegenden Zeichnungen näher erläutert.

Hierbei zeigen:
- Fig. 1: ein Bestrahlungsgerät nach dem Stand der Technik
- Fig. 2: ein Bestrahlungsgerät gemäß einer Ausführungsform der vorliegenden Erfindung, bei dem zwischen T12-Niederdruckstrahlern mit UVA-Spektrum jeweils T5-Niederdruckstrahler mit einem vorbestimmten UVB-Anteil angeordnet sind
- Fig. 3: eine alternative Ausführungsform eines Bestrahlungsgerätes, bei dem alternierend T12-Niederdruckstrahler und T5-Niederdruckstrahler angeordnet sind, wobei die T12-Niederdruckstrahler ein UVA-Spektrum und die T5-Niederdruckstrahler einen vorbestimmten UVB-Anteil aufweisen
- Fig. 4: eine erste Ausführungsform für eine Anordnung der Niederdruckstrahler in einem Bestrahlungsfeld
- Fig. 5: eine zweite Prinzipskizze zur Erläuterung einer alternativen Anordnungsmöglichkeit der Niederdruckstrahler in einem Bestrahlungsfeld

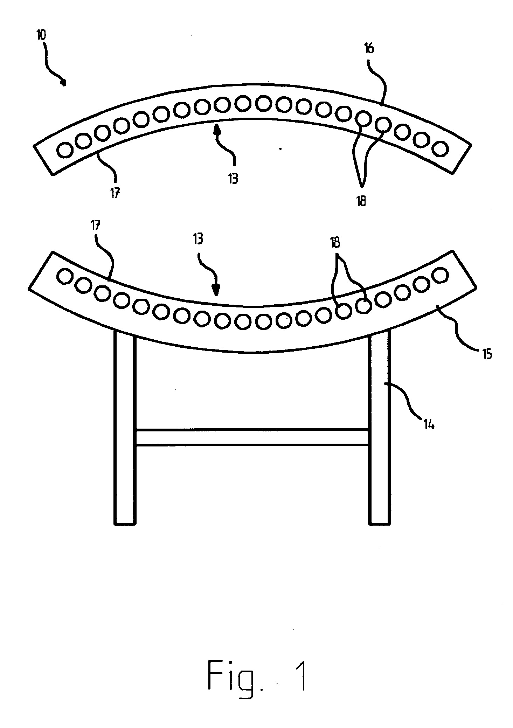

In Fig. 1 ist ein Bestrahlungsgerät 10 nach dem Stand der Technik schematisch dargestellt. Das Bestrahlungsgerät 10 nach dem Stand der Technik umfasst zwei Gehäuse 15, 16, die an ihren einander zugewandten Seiten jeweils mit einer UV-durchlässigen Scheibe 17 versehen sind. Das in Aufstellposition untere Gehäuse 15 definiert eine Liegefläche und kann auf einem rahmenartigen Gestell 14 befestigt sein. Das in Aufstellposition obere Gehäuse 16 definiert einen so genannten "Himmel" und kann am unteren Gehäuse 15 beispielsweise über ein Scharnier befestigt sein. Alternativ könnte das obere Gehäuse 16 auch anderweitig, beispielsweise direkt an einer Decke eines Raumes befestigt sein.

Sowohl im unteren Gehäuse 15 wie auch im oberen Gehäuse 16 sind eine Mehrzahl von Niederdruckstrahlern 18 nach dem Stand der Technik parallel zueinander angeordnet und definieren jeweils ein Bestrahlungsfeld 13. Die Haupt-Abstrahlrichtung des durch die Niederdruckstrahler 18 definierten Bestrahlungsfeldes 13 im unteren Gehäuse 15 ist vertikal nach oben in Richtung auf das obere Gehäuse 16 gerichtet. In gleicher Weise ist die Haupt-Abstrahlrichtung der das Bestrahlungsfeld 13 des oberen Gehäuses 16 definierenden Niederdruckstrahler 18 vertikal nach unten in Richtung auf das untere Gehäuse 15 gewandt.

Auch die Bestrahlungsgeräte 10 nach dem Stand der Technik versuchen eine möglichst homogene Abstrahlcharakteristik der Strahlungsfelder 13 zu erzielen, um eine möglichst gleichmäßige Bestrahlung eines menschlichen Körpers zu ermöglichen. Gleichzeitig bestand jedoch der Wunsch, Bestrahlungsgeräte zu konzipieren, bei denen das Verhältnis der abgestrahlten Leistung im UVA- zum UVB-Bereich einstellbar ist. Die mit einstellbarer UVB- zu UVA-Leistung vorgeschlagenen Bestrahlungsgeräte nach dem Stand der Technik erfüllen jedoch nicht die anwendungsseitig gegebenen Anforderungen an eine relativ homogene Abstrahlung.

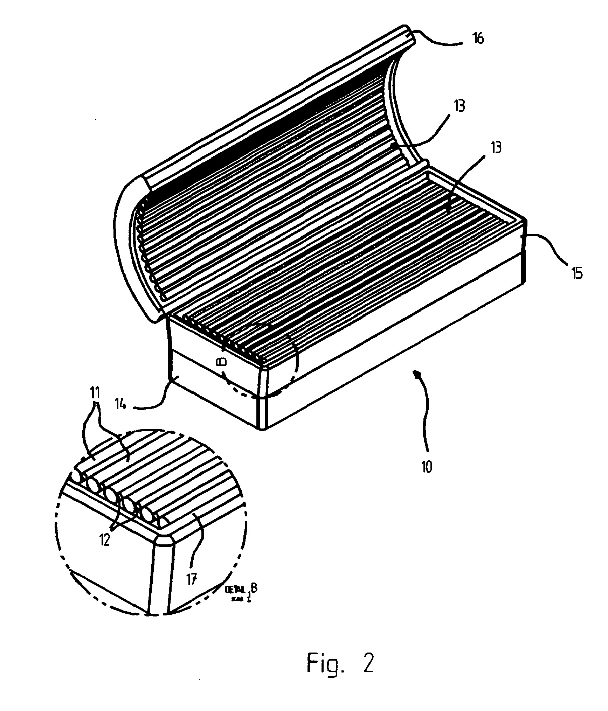

In Fig. 2 ist eine Ausführungsform eines Bestrahlungsgerätes 10 nach der vorliegenden Erfindung veranschaulicht. Ein oberes Gehäuse 16 ist schwenkbar an einem unteren Gehäuse 15 angelenkt. Das untere Gehäuse 15 ist auf einem rahmenartigen Gestell 14 abgestützt. Sowohl im unteren Gehäuse 15 als auch im oberen Gehäuse 16 sind eine Mehrzahl von Niederdruckstrahlern 11 eines ersten Typs A sowie eine Mehrzahl von Niederdruckstrahlern 12 eines zweiten Typs B alternierend zueinander, in paralleler Ausrichtung (vgl. auch Detailansicht A) angeordnet.

Die Niederdruckstrahler des ersten Typs A sind dabei gemäß der hier konkret dargestellten Ausführungsform als T12-Niederdruckstrahler bzw. T12-Niederdruck-Leuchtstoff-UV-Strahler ausgebildet und zur Abstrahlung eines UVA-Spektrums ausgelegt. T12-Niederdruck-Leuchtstoff-UV-Strahler sind an sich bekannt und weisen einen Durchmesser von 12/8 Inch = 38,1 mm auf. Derart vergleichsweise dicke Niederdruckstrahler eigenen sich an sich gut, um ein Bestrahlungsfeld 13 zu belegen, da hier mit vergleichsweise kostengünstigen Strahlern eine breite Fläche überdeckt werden kann.

Das Bestrahlungsfeld 13 innerhalb des unteren Gehäuses 15 kann beispielsweise eine Länge von 200 cm bei einer Breite von 70 cm aufweisen. Das Bestrahlungsfeld 13 des oberen Gehäuses 16 weist eine entsprechende Länge auf und kann wegen der gewölbten Ausbildung eine Bogenlänge von etwas mehr als 70 cm aufweisen. Innerhalb des Bestrahlungsfeldes 13 des oberen Gehäuses 16 kann randseitig (an einem Kopfende) noch ein Bereich für ein Gesichtsbräunungsfeld vorgesehen sein, das beispielsweise durch eine Mehrzahl von Hochdruck-UV-Strahlern oder T5-Strahlern gebildet sein kann.

Weiter kann innerhalb des Bestrahlungsfeldes 13 auch ein Gesichtsbräuner der aus kurzen T5-Niederdruckstrahlern oder aus Hochdruck-Quarzstrahlern gebildet ist, integriert sein. Diese Strahler des Gesichtsbräuners können separat vom Hauptfeld angesteuert und in der gewünschten Strahlenstärke eingestellt werden.

Bei der hier dargestellten Ausführungsform sind jedoch zwischen den T12-Niederdruckstrahlern 11 des ersten Typs A noch Niederdruckstrahler 12 des zweiten Typs B, die hier konkret als T5-Niederdruck-Leuchtstoff-UV-Strahler ausgebildet sind, angeordnet. Die Niederdruckstrahler 12 des zweiten Typs B, die hier konkret als T5-Strahler ausgebildet sind, strahlen auch einen bestrahlungswirksamen UVB-Anteil ab. T5-Niederdruck-Leuchtstoff-UV-Strahler sind ebenfalls an sich schon bekannt, wenngleich nur in vergleichsweise kurzen Längen. T5-Niederdruck-Leuchtstoff-UV-Strahler weisen einen Durchmesser von 5/8 Inch, entsprechend etwa 15,9 mm auf. Wie aus der Detailansicht A besonders gut ersichtlich, sind die Niederdruckstrahler 12 des zweiten Typs B hier in Abstrahlrichtung gegenüber den Niederdruckstrahlern 11 des ersten Typs A derart versetzt, dass sie an ihrer der oberen Gehäusehälfte 16 zugewandten Seite bzw. an ihrer der Abstrahlrichtung zugewandten Seite im wesentlichen bündig abschließen. In entsprechender Weise ist dies im Bestrahlungsfeld 13 innerhalb des oberen Gehäuses 16 gelöst. Auch hier sind die Niederdruckstrahler 12 des zweiten Typs B gegenüber den Niederdruckstrahlern 11 des ersten Typs A derart in Abstrahlrichtung versetzt, dass sie an ihrer dem unteren Gehäuse 15 zugewandten Seite bzw. an ihrer der Abstrahlrichtung zugewandten Seite im wesentlichen bündig abschließen.

Die Niederdruckstrahler 11 des ersten Typs A und/oder die Niederdruckstrahler 12 des zweiten Typs B können einen in ihren Kolben integrierten Reflektor, der beispielsweise als eine Al₂O₃-Schicht ausgebildet sein kann, umfassen. Die Al₂O₃-Schicht kann beispielsweise durch zweimaliges Beschichten innen über ca. die Hälfte des Kolbenumfangs der Niederdruckstrahler 11 des ersten Typs A und/oder der Niederdruckstrahler 12 des zweiten Typs B ausgebildet werden. Alternativ oder zusätzlich kann auch jeweils im Gehäuse 15, 16 ein Reflektor hinter den Niederdruckstrahlern 11, 12, also an der der Abstrahlrichtung abgewandten Seite vorgesehen sein. Auch diese im Gehäuse 15, 16 integrierten Reflektoren können auf metallischer Basis, beispielsweise aus Aluminium, ausgebildet sein.

Die Niederdruckstrahler 12 des zweiten Typs B können typischerweise bezogen auf ihre gesamte erythemwirksame UV-Leistung ca. 5% bis nahezu 100% an UVB-Leistung abgeben.

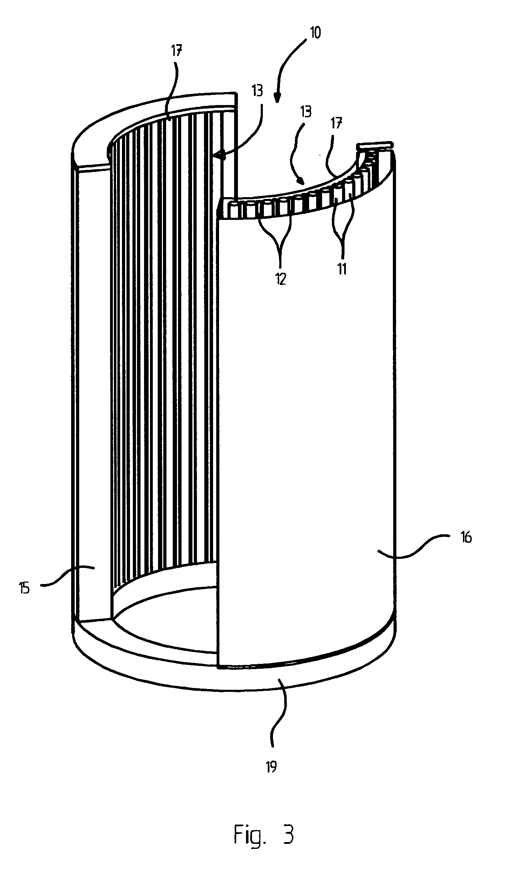

In Fig. 3 ist eine nochmals abgewandelte Ausführungsform eines Bestrahlungsgerätes veranschaulicht. Bei der dort dargestellten Ausführungsform sind zwei Standgehäuse 15', 16' auf einem ringförmigen Fußelement 19 befestigt. Auch hier weisen die Standgehäuse 15', 16' an ihren einander zugewandten Seiten eine UV-durchlässige Scheibe 17 auf. Zwischen den Standgehäusen 15', 16' ist seitlich eine Beabstandung vorgesehen, so dass ein Benutzer seitlich eintreten und in stehender Position etwa in der Mitte des ringförmigen Fußelements 19 positioniert sich mit UV-Licht ausgehend von den Standgehäusen 15, 16 bestrahlen lassen kann.

Zu diesem Zweck weisen die Standgehäuse 15', 16' ebenfalls eine Mehrzahl von Niederdruckstrahlern 11 des ersten Typs A sowie eine Mehrzahl von Niederdruckstrahlern 12 des zweiten Typs B auf, die alternierend zueinander in paralleler Ausrichtung von der UV-durchlässigen Scheibe 17 abgedeckt im Standgehäuse 15', 16' angeordnet sind. Auch hier können die Niederdruckstrahler 12 des zweiten Typs B in Abstrahlrichtung, (das heißt jeweils in Richtung auf das gegenüberliegend angeordnete Standgehäuse 16', 15') gegenüber den Niederdruckstrahlern 11 des ersten Typs A versetzt angeordnet sein derart, dass sie an ihrer der Abstrahlrichtung zugewandten Seite bzw. in Richtung auf das gegenüberliegende Standgehäuse 16', 15' im Wesentlichen bündig abschließen.

Ähnlich wie in der Ausführungsform nach Fig. 2 können auch hier die Niederdruckstrahler 11, 12 mit einem integrierten Reflektor, beispielsweise einer Al₂O₃-Schicht, ausgestattet sein und/oder es kann ein Reflektor hinter den Niederdruckstrahlern 11, 12 im Standgehäuse 15', 16' integriert sein.

Sowohl in der Ausführungsform nach Fig. 2 als auch in der Ausführungsform nach Fig. 3 können die Niederdruckstrahler 11, 12 mit einer Länge von bis zu zwei Metern oder darüber ausgebildet sein. T5-Niederdruck-Leuchtstoff-UV-Strahler in einer Länge von 180 cm bzw. 6 ft oder mehr sind im Stand der Technik bislang nicht bekannt, so dass eine solche Lichtquelle, insbesondere zur Abstrahlung von UV-Licht und/oder insbesondere zum Einsatz in UV-Bestrahlungsgeräte auch unabhängig beansprucht wird.

Selbstverständlich ist es im Rahmen der Erfindung aber auch möglich, nicht über das gesamte Bestrahlungsfeld 13 durchgehende Niederdruckstrahler 11 des ersten Typs A oder Niederdruckstrahler 12, des zweiten Typs B einzusetzen. Gerade bei als T5-Niederdruck-Leuchtstoff-UV-Strahlern ausgebildeten Niederdruckstrahlern 12 des zweiten Typs B könnten herkömmliche T5-Röhren von 4 ft und 2 ft aneinandergesetzt werden, um eine Länge von 6 ft zu erreichen.

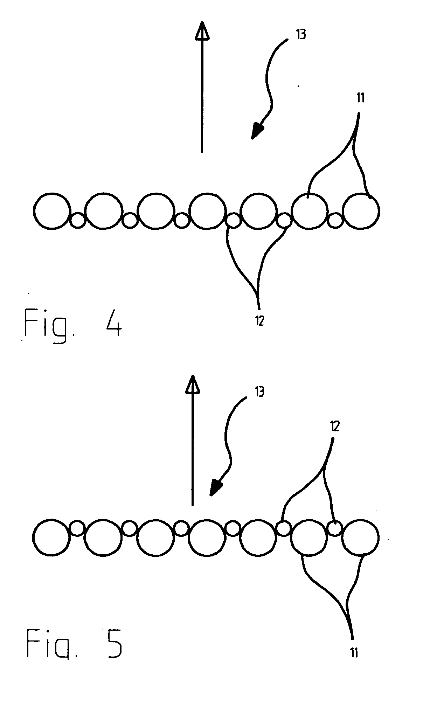

In den Fig. 4 und 5 sind zwei mögliche Anordnungen der Niederdruckstrahler 12 des zweiten Typs B relativ zu den Niederdruckstrahlern 11 des ersten Typs A veranschaulicht. Dabei sind in der Prinzipskizze in Fig. 4 die Niederdruckstrahler 12 des ersten Typs B gegenüber den Niederdruckstrahlern 11 des ersten Typs A entgegen der Abstrahlrichtung nach hinten versetzt und zwar so, dass sie an ihrer der Abstrahlrichtung abgewandten Seite im wesentlichen bündig abschließen.

Wie aus Fig. 4 ersichtlich, ergibt sich hierdurch ein Bestrahlungsfeld 13, bei dem in Projektion auf die Abstrahlrichtung zwischen den Niederdruckstrahlern 11 und 12 praktisch keine Lücken entstehen, also das gesamte Bestrahlungsfeld 13 durch Niederdruckstrahler 11 oder 12 zumindest im wesentlichen vollflächig abgedeckt ist.

Bei der Anordnung nach Fig. 5 lässt sich ebenfalls eine im wesentlichen vollflächige Abdeckung des Bestrahlungsfeldes 13 durch Niederdruckstrahler 11 oder 12 erreichen, wobei hier die Niederdruckstrahler 12 des zweiten Typs B gegenüber den Niederdruckstrahlern 11 des ersten Typs A in Richtung auf die Abstrahlrichtung versetzt angeordnet sind, wie dies bereits bei Erläuterung der Ausführungsform nach Fig. 2 und 3 dargelegt wurde, nämlich hier konkret so, dass sie an ihrer der Abstrahlrichtung zugewandten Seite im Wesentlichen bündig abschließen.

### Bezugszeichenliste

- 10: Bestrahlungsgerät
- 11: Niederdruckstrahler (Typ A)
- 12: Niederdruckstrahler (Typ B)
- 13: Bestrahlungsfeld
- 14: Rahmenartiges Gestell
- 15, 16: Gehäuse
- 15', 16': Standgehäuse
- 17: UV-durchlässige Scheibe
- 18: Niederdruckstrahler (nach dem Stand der Technik)
- 19: Ringförmiges Fußelement

## Patentansprüche

1. Bestrahlungsgerät zur Bestrahlung eines menschlichen Körpers mit mindestens zwei Typen röhrenförmiger, UVA und/oder UVB-Strahlung abgebender Niederdruckstrahler (11, 12), die alternierend, parallel zueinander zumindest einen Teilbereich eines Bestrahlungsfeldes (13) oder das komplette Bestrahlungsfeld (13) des Bestrahlungsgerätes überdeckend, dicht aneinander liegend angeordnet sind,
wobei die Niederdruckstrahler (12) des zweiten Typs B gegenüber den Niederdruckstrahlern (11) des ersten Typs A eine höhere UVB-Abstrahlung aufweisen,
wobei der Durchmesser der Niederdruckstrahler (12) des zweiten Typs B kleiner als der Durchmesser der Niederdruckstrahler (11) des ersten Typs A ist und wobei die Niederdruckstrahler (11) des ersten Typs A und die Niederdruckstrahler (12) des zweiten Typs B getrennt angesteuert sind,
**dadurch gekennzeichnet, dass**
die Niederdruckstrahler des zweiten Typs B als reine UVB-Lampen ausgebildet sind und die Niederdruckstrahler (11) des ersten Typs (A) als reine UVA-Lampen ausgebildet sind;
das Verhältnis des Durchmessers der Niederdruckstrahler (12) des zweiten Typs B und des Durchmessers der Niederdruckstrahler (11) des ersten Typs A etwa 5:12 beträgt,
die Niederdruckstrahler (11) des ersten Typs A und die Niederdruckstrahler (12) des zweiten Typs B in ihrer erythemwirksamen Leistung getrennt einstellbar sind, und
die Niederdruckstrahler (12) zumindest des zweiten Typs B, vorzugsweise auch die Niederdruckstrahler (11) des ersten Typs A, eine Länge ≥ 120 cm, vorzugsweise ≥ 180 cm, aufweisen.

2. Bestrahlungsgerät nach Anspruch 1,
**dadurch gekennzeichnet,**
**dass** die Niederdruckstrahler (11) des ersten Typs A durch T12-Röhren, also Niederdruckstrahler mit einem Durchmesser von 12/8 Inch = 38,1 mm gebildet sind.

3. Bestrahlungsgerät nach einem der Ansprüche 1 oder 2,
**dadurch gekennzeichnet,**
**dass** die Niederdruckstrahler (12) des zweiten Typs B durch T5-Röhren, also Niederdruckstrahler mit einem Durchmesser von 5/8 Inch, entsprechend 15,9 mm gebildet sind.

4. Bestrahlungsgerät nach einem der Ansprüche 1 bis 3,
**dadurch gekennzeichnet,**
**dass** die Niederdruckstrahler (11) des ersten Typs A und die Niederdruckstrahler (12) des zweiten Typs B im Bestrahlungsfeld (13) insofern dicht aneinander liegen, als sie nicht mehr als ein viertel Lampendurchmesser der Niederdruckstrahler (11) des ersten Typs A, bevorzugt nicht mehr als ein viertel Lampendurchmesser der Niederdruckstrahler (12) des zweiten Typs B beabstandet sind.

5. Bestrahlungsgerät nach einem der Ansprüche 1 bis 4,
**dadurch gekennzeichnet,**
**dass** das Bestrahlungsfeld (13) in Projektion auf die Haupt-Abstrahlrichtung im Wesentlichen vollständig, mindestens aber zu 95% abwechselnd durch Niederdruckstrahler (11) des ersten Typs A und durch Niederdruckstrahler (12) des zweiten Typs B überdeckt ist.

6. Bestrahlungsgerät nach einem der Ansprüche 1 bis 5,
**dadurch gekennzeichnet,**
**dass** die Niederdruckstrahler (12) des zweiten Typs B gegenüber den Niederdruckstrahlern (11) des ersten Typs A geringfügig in Richtung der Haupt-Abstrahlrichtung versetzt sind, dergestalt, dass sie an ihrer der Haupt-Abstrahlrichtung zugewandten Seite zumindest im wesentlichen bündig abschliessen.

7. Bestrahlungsgerät nach einem der Ansprüche 1 bis 5,
**dadurch gekennzeichnet,**
**dass** die Niederdruckstrahler (12) des zweiten Typs B gegenüber den Niederdruckstrahlern (11) des ersten Typs A geringfügig in Richtung entgegen der Haupt-Abstrahlrichtung versetzt sind, dergestalt, dass sie an ihrer der Hauptabstrahlrichtung abgewandten Seite zumindest im Wesentlichen bündig zueinander abschliessen.

8. Bestrahlungsgerät nach einem der Ansprüche 1 bis 7,
**dadurch gekennzeichnet,**
**dass** die Niederdruckstrahler (11) des ersten Typs A eine Länge von mindestens 60 cm, vorzugsweise von mindestens 150 cm, weiter vorzugsweise von mindestens 180 cm, insbesondere von mindestens 200 cm aufweisen.

9. Bestrahlungsgerät nach einem der Ansprüche 1 bis 8,
**dadurch gekennzeichnet,**
**dass** etwa 15% bis 35%, bevorzugtermassen etwa 30% des Bestrahlungsfeldes (13) durch Niederdruckstrahler (12) des zweiten Typs B überdeckt ist.

10. Bestrahlungsgerät nach einem der Ansprüche 1 bis 9,
**dadurch gekennzeichnet,**
**dass** etwa 65% bis 75%, bevorzugtermassen etwa 70% des Bestrahlungsfeldes (13) durch Niederdruckstrahler (11) des ersten Typs A überdeckt ist.

11. Bestrahlungsgerät nach einem der Ansprüche 1 bis 10,
**dadurch gekennzeichnet,**
**dass** im Bestrahlungsfeld (13) ein Gesichtsbräuner integriert ist, der vorzugsweise eine Mehrzahl von T5-Niederdruckstrahlern oder eine Mehrzahl von Hochdruck-Quarzstrahlern umfasst, wobei der Gesichtsbräuner bzw. dessen Strahler separat von den Niederdruckstrahlern (11, 12) des verbleibenden Bestrahlungsfeldes (13) angesteuert und in der gewünschten Strahlenstärke eingestellt werden können.

12. Verwendung eines Bestrahlungsgerätes nach einem der Ansprüche 1 bis 11 für die kosmetische Bräunung eines menschlichen Körpers.

13. Verfahren zum Betreiben eines Bestrahlungsgerätes, insbesondere zur Bestrahlung eines menschlichen Körpers mit mindestens zwei Typen röhrenförmiger, UVA und/oder UVB-Strahlung abgebender Niederdruckstrahler (11, 12), die parallel zueinander zumindest einen Teilbereich eines Bestrahlungsfeldes (13) oder das komplette Bestrahlungsfeld (13) des Bestrahlungsgerätes überdeckend dicht aneinander liegend angeordnet sind, wobei die Niederdruckstrahler (12) des zweiten Typs B gegenüber den Niederdruckstrahlern (11) des ersten Typs A eine höhere UVB-Abstrahlung aufweisen, und
wobei die Niederdruckstrahler (11) des ersten Typs A und die Niederdruckstrahler (12) des zweiten Typs B getrennt angesteuert werden,
wobei zur Einstellung der UVB-Leistung die Leistung der Niederdruckstrahler (12) des zweiten Typs B gegenüber einer vorzugsweise konstant gehaltenen Leistung der Niederdruckstrahler (11) des ersten Typs A erhöht bzw. reduziert wird,
wobei eine Bestrahlungsstärke von UVA- und UVB-Strahlung durch eine Lampenleistung eingestellt wird,
**dadurch gekennzeichnet, dass**
der im Bestrahlungsfeld (13) durch Niederdruckstrahler des ersten Typs A belegte Flächenanteil mindestens doppelt so gross ist wie der durch Niederdruckstrahler (12) des zweiten Typs B belegte Flächenanteil, wobei die Niederdruckstrahler (12) des zweiten Typs B einen gegenüber dem Durchmesser der Niederdruckstrahler (11) des ersten Typs A reduzierten Durchmesser aufweisen,
die Niederdruckstrahler des zweiten Typs B als reine UVB-Lampen ausgebildet sind und die Niederdruckstrahler (11) des ersten Typs (A) als reine UVA-Lampen ausgebildet sind;
die Niederdruckstrahler (11) des ersten Typs A und die Niederdruckstrahler (12) des zweiten Typs B in ihrer erythemwirksamen Leistung getrennt eingestellt werden, und
die Niederdruckstrahler (12) zumindest des zweiten Typs B, vorzugsweise auch die Niederdruckstrahler (11) des ersten Typs A, eine Länge ≥ 120 cm, vorzugsweise ≥ 180 cm, aufweisen.

14. Verfahren nach Anspruch 13,
**dadurch gekennzeichnet,**
**dass** die Einstellung der UVB-Leistung der Niederdruckstrahler (12) des zweiten Typs B in Abhängigkeit des Hauttyps einer zu bestrahlenden Person vorgenommen wird.

15. Verfahren nach Anspruch 14,
**dadurch gekennzeichnet,**
**dass** der Hauttyp einer zu bestrahlenden Person automatisch ermittelt wird.

## Claims

1. An irradiation device for irradiating a human body with at least two types of tubular, UVA and/or UVB radiation-emitting low-pressure lamps (11, 12), which are arranged close to one another in an alternating manner parallel to one another covering at least a portion of an irradiation field (13) or the entire irradiation field (13) of the irradiation device,
wherein the low-pressure lamps (12) of the second type B have a higher UVB emission compared to the low-pressure lamps (11) of the first type A,
wherein the diameter of the low-pressure lamps (12) of the second type B is smaller than the diameter of the low-pressure lamps (11) of the first type A, and
wherein the low-pressure lamps (11) of the first type A and the low-pressure lamps (12) of the second type B are controlled separately,
**characterised in that**
the low-pressure lamps of the second type B are configured as pure UVB bulbs and the low-pressure lamps (11) of the first type (A) are configured as pure UVA bulbs;
the ratio of the diameter of the low-pressure lamps (12) of the second type B to the diameter of the low-pressure lamps (11) of the first type A is approximately 5:12,
the low-pressure lamps (11) of the first type A and the low-pressure lamps (12) of the second type B are separately adjustable in terms of their erythema-effective output, and the low-pressure lamps (12) of at least the second type B, preferably also the low-pressure lamps (11) of the first type A, have a length ≥ 120cm, and preferably ≥ 180cm.

2. The irradiation device according to Claim 1,
**characterised in that**
the low-pressure lamps (11) of the first type A are formed by T12 tubes, i.e. low-pressure lamps with a diameter of 12/8 of an inch = 38.1 mm.

3. The irradiation device according to one of claims 1 or 2,
**characterised in that**
the low-pressure lamps (12) of the second type B are formed by T5 tubes, i.e. low-pressure lamps with a diameter of 5/8 of an inch, corresponding to 15.9mm.

4. The irradiation device according to one of claims 1 to 3,
**characterised in that**
the low-pressure lamps (11) of the first type A and the low-pressure lamps (12) of the second type B are situated close to one another in the irradiation field (13) insofar that they are not spaced apart by more than a quarter bulb diameter of the low-pressure lamps (11) of the first type A, preferably not by more than a quarter bulb diameter of the low-pressure lamps (12) of the second type B.

5. The irradiation device according to one of claims 1 to 4,
**characterised in that**
the irradiation field (13) in projection onto the main emission direction is covered substantially completely, but at least up to 95% alterrnately by low-pressure lamps (11) of the first type A and by low-pressure lamps (12) of the second type B.

6. The irradiation device according to one of claims 1 to 5,
**characterised in that**
the low-pressure lamps (12) of the second type B are slightly offset towards the main emission direction compared to the low-pressure lamps (11) of the first type A in such a way that they are substantially flush at their side facing the main emission direction.

7. The irradiation device according to one of claims 1 to 5,
**characterised in that**
the low-pressure lamps (12) of the second type B are slightly offset against the main emission direction compared to the low-pressure lamps (11) of the first type A in such a way that they are substantially flush at their side facing away from the main emission direction.

8. The irradiation device according to one of claims 1 to 7,
**characterised in that**
the low-pressure lamps (11) of the first type A have a length of at least 60cm, preferably of at least 150cm, more preferably of at least 180cm, particularly of at least 200cm.

9. The irradiation device according to one of claims 1 to 8,
**characterised in that**
approximately 15% to 35%, preferably approximately 30% of the irradiation field (13) is covered by low-pressure lamps (12) of the second type B.

10. The irradiation device according to one of claims 1 to 9,
**characterised in that**
approximately 65% to 75%, preferably approximately 70% of the irradiation field (13) is covered by low-pressure lamps (11) of the first type A.

11. The irradiation device according to one of claims 1 to 10,
**characterised in that**
in the irradiation field (13) there is integrated a face tanner, which preferably includes a plurality of T5-low-pressure lamps or a plurality of high-pressure quartz lamps, wherein the face tanner or its lamps can be controlled separately from the low-pressure lamps (11, 12) of the remaining irradiation field (13) and can be adjusted into the desired radiation strength.

12. A use of an irradiation device according to one of claims 1 to 11 for the cosmetic tanning of a human body.

13. A method for operating an irradiation device, in particular for irradiating a human body with at least two types of tubular, UVA and/or UVB radiation-emitting low-pressure lamps (11, 12), which are arranged close to one another parallel to one another covering at least a portion of an irradiation field (13) or the entire irradiation field (13) of the irradiation device,
wherein the low-pressure lamps (12) of the second type B have a higher UVB emission compared to the low-pressure lamps (11) of the first type A, and
wherein the low-pressure lamps (11) of the first type A and the low-pressure lamps (12) of the second type B are controlled separately,
wherein, to adjust the UVB output, the output of the low-pressure lamps (12) of the second type B is increased or reduced compared to an output of the low-pressure lamps (11) of the first type A which is preferably kept constant,
wherein an irradiation strength of UVA and UVB radiation is adjusted by a bulb output,
**characterised in that**
the surface percentage occupied in the irradiation field (13) by low-pressure lamps of the first type A is at least twice as large as the surface percentage occupied by low-pressure lamps (12) of the second type B, wherein the low-pressure lamps (12) of the second type B have a reduced diameter compared to the diameter of the low-pressure lamps (11) of the first type A;
the low-pressure lamps of the second type B are configured as pure UVB bulbs and the low-pressure lamps (11) of the first type (A) are configured as pure UVA bulbs;
the low-pressure lamps (11) of the first type A and the low-pressure lamps (12) of the second type B are separately adjusted in terms of their erythema-effective output, and the low-pressure lamps (12) of at least the second type B, preferably also the low-pressure lamps (11) of the first type A, have a length ≥ 120cm, preferably ≥ 180cm.

14. The method according to Claim 13,
**characterised in that**
the adjustment of the UVB output of the low-pressure lamps (12) of the second type B is carried out in accordance with the skin type of a person to be irradiated.

15. The method according to Claim 14,
**characterised in that**
the skin type of a person to be irradiated is detected automatically.

## Revendications

1. Dispositif d'irradiation destiné à l'irradiation d'un corps humain avec au moins deux types d'émetteurs basse pression (11, 12) tubulaires, émettant un rayonnement UVA et/ou UVB, agencés de manière alternée, parallèles et proches les uns des autres et couvrant au moins une partie d'un champ d'irradiation (13) ou la totalité du champ d'irradiation (13) du dispositif d'irradiation,
dans lequel les émetteurs basse pression (12) du second type B émettent un rayonnement UVB supérieur à celui des émetteurs basse pression (11) du premier type A,
dans lequel le diamètre des émetteurs basse pression (12) du second type B est plus petit que le diamètre des émetteurs basse pression (11) du premier type A et dans lequel les émetteurs basse pression (11) du premier type A et les émetteurs basse pression (12) du second type B sont commandés séparément,
**caractérisé en ce que**
les émetteurs basse pression du second type B sont formés comme des lampes uniquement à UVB et les émetteurs basse pression (11) du premier type A sont formés comme des lampes uniquement à UVA ;
le rapport entre le diamètre des émetteurs basse pression (12) du second type B et le diamètre des émetteurs basse pression (11) du premier type A est d'environ 5:12, les émetteurs basse pression (11) du premier type A et les émetteurs basse pression (12) du second type B sont réglables séparément en ce qui concerne leur puissance efficace contre l'érythème, et
les émetteurs basse pression (12) au moins du second type B, de préférence également les émetteurs basse pression (11) du premier type A, présentent une longueur ≥ 120 cm, de préférence ≥ 180 cm.

2. Dispositif d'irradiation selon la revendication 1,
**caractérisé en ce que**
les émetteurs basse pression (11) du premier type A sont formés par des tubes T12, à savoir des émetteurs basse pression avec un diamètre de 12/8 de pouce = 38,1 mm.

3. Dispositif d'irradiation selon l'une des revendications 1 ou 2,
**caractérisé en ce que**
les émetteurs basse pression (12) du second type B sont formés par des tubes T5, à savoir des émetteurs basse pression avec un diamètre de 5/8 de pouce, correspondant à 15,9 mm.

4. Dispositif d'irradiation selon l'une des revendications 1 à 3,
**caractérisé en ce que**
les émetteurs basse pression (11) du premier type A et les émetteurs basse pression (12) du second type B sont situés proches les uns des autres dans le champ d'irradiation (13), de manière à ne pas être espacés de plus d'un quart de diamètre de lampe des émetteurs basse pression (11) du premier type A, de préférence pas plus d'un quart de diamètre de lampe des émetteurs basse pression (12) du second type B.

5. Dispositif d'irradiation selon l'une des revendications 1 à 4,
**caractérisé en ce que**
le champ d'irradiation (13), en projection le long de la direction de rayonnement principale, est substantiellement complètement couvert, ou au moins à 95 %, en alternance, par les émetteurs basse pression (11) du premier type A et par les émetteurs basse pression (12) du second type B.

6. Dispositif d'irradiation selon l'une des revendications 1 à 5,
**caractérisé en ce que**
les émetteurs basse pression (12) du second type B sont légèrement décalés par rapport aux émetteurs basse pression (11) du premier type A dans la direction de rayonnement principale, de sorte qu'ils se terminent au moins essentiellement en affleurement sur leur côté faisant face à la direction de rayonnement principale.

7. Dispositif d'irradiation selon l'une des revendications 1 à 5,
**caractérisé en ce que**
les émetteurs basse pression (12) du second type B sont légèrement décalés par rapport aux émetteurs basse pression (11) du premier type A dans la direction opposée à la direction de rayonnement principale, de sorte qu'ils se terminent, au moins essentiellement en affleurement sur leur côté opposé à la direction de rayonnement principale.

8. Dispositif d'irradiation selon l'une des revendications 1 à 7,
**caractérisé en ce que**
les émetteurs basse pression (11) du premier type A présentent une longueur d'au moins 60 cm, de préférence au moins 150 cm, de manière plus préférée au moins 180 cm, en particulier au moins 200 cm.

9. Dispositif d'irradiation selon l'une des revendications 1 à 8,
**caractérisé en ce que**
environ 15 % à 35 %, de préférence environ 30 %, du champ d'irradiation (13) sont couverts par les émetteurs basse pression (12) du second type B.

10. Dispositif d'irradiation selon l'une des revendications 1 à 9,
**caractérisé en ce que**
environ 65 % à 75 %, de préférence environ 70 %, du champ d'irradiation (13) sont recouverts par les émetteurs basse pression (11) du premier type A.

11. Dispositif d'irradiation selon l'une des revendications 1 à 10,
**caractérisé en ce que**
dans le champ d'irradiation (13) est intégré un système de bronzage du visage, lequel comprend de préférence une pluralité d'émetteurs basse pression T5 ou une pluralité d'émetteurs à quartz haute tension, le système de bronzage du visage ou ses émetteurs pouvant être commandés séparément des émetteurs basse pression (11, 12) du champ d'irradiation (13) restant et peuvent être réglés sur la puissance de rayonnement souhaitée.

12. Utilisation d'un dispositif d'irradiation selon l'une des revendications 1 à 11 pour le bronzage d'un corps humain à des fins cosmétiques.

13. Procédé d'exploitation d'un dispositif d'irradiation, en particulier pour l'irradiation d'un corps humain avec au moins deux types d'émetteurs basse pression (11, 12) tubulaires, émettant un rayonnement UVA et/ou UVB, agencés de manière alternée, parallèles et proches les uns des autres et couvrant au moins une partie d'un champ d'irradiation (13) ou la totalité du champ d'irradiation (13) du dispositif d'irradiation, dans lequel les émetteurs basse pression (12) du second type B émettent un rayonnement UVB supérieur à celui des émetteurs basse pression (11) du premier type A, et dans lequel les émetteurs basse pression (11) du premier type A et les émetteurs basse pression (12) du second type B sont commandés séparément,
dans lequel, pour le réglage de la puissance en UVB, la puissance des émetteurs basse pression (12) du second type B est augmentée ou diminuée par rapport à une puissance maintenue de préférence constante des émetteurs basse pression (11) du premier type A,
dans lequel une puissance de rayonnement du rayonnement UVA et UVB est réglée par le biais d'une puissance de lampe,
**caractérisé en ce que**
la portion de surface dans le champ d'irradiation (13) occupée par les émetteurs basse pression du premier type A est au moins deux fois plus grande que la portion de surface occupée par les émetteurs basse pression (12) du second type B, dans lequel les émetteurs basse pression (12) du second type B présentent un diamètre réduit par rapport au diamètre des émetteurs basse pression (11) du premier type A,
les émetteurs basse pression du second type B sont formés comme des lampes uniquement à UVB et les émetteurs basse pression (11) du premier type (A) sont formés comme des lampes uniquement à UVA ;
les émetteurs basse pression (11) du premier type A et les émetteurs basse pression (12) du second type B sont réglés séparément en ce qui concerne leur puissance contre l'érythème, et
les émetteurs basse pression (12) au moins du second type B, de préférence également les émetteurs basse pression (11) du premier type A, présentent une longueur ≥ 120 cm, de préférence ≥ 180 cm.

14. Procédé selon la revendication 13,
**caractérisé en ce que**
le réglage de la puissance en UVB des émetteurs basse pression (12) du second type B est réalisé en fonction du type de peau d'une personne à irradier.

15. Procédé selon la revendication 14,
**caractérisé en ce que**
le type de peau d'une personne à irradier est détecté de manière automatique.
